# EUROPEAN PATENT APPLICATION

(11) **EP 2 497 844 A1**
(43) Date of publication of application: **12.09.2012**
(21) Application number: 11157636.9
(22) Date of filing: 10.03.2011
(51) Int. Cl.: C23F 11/14, C09K 8/54, E21B 41/02, C23F 11/04, C07C 219/06, C11D 1/62, C11D 1/645

(54) **Quaternary ammonium esters (Esterquats) containing composition for inhibiting corrosion of metal surface**

(71) Applicant: KAO CORPORATION, S.A., 08210 Barbera del Vallés - Barcelona (ES)
(72) Inventor: Bueno Perisé, Agusti, Barcelona (ES); Lozano Salvatella, Luis, 08023, Barcelona (ES); Mundó Blanch, Miquel, 08500, VIC (Barcelona) (ES); Riaza Martinez, Joan Antoni, 17230, Palamós (Girona) (ES)
(74) Representative: HOFFMANN EITLE

(57) **Abstract**

The present invention relates to a composition that is suitable for use as a corrosion inhibitor to prevent corrosion, particularly on metallic devices for the recovery and transportation of hydrocarbons in oil production and processing. In particular, the present invention relates to quaternary ammonium compositions found to be effective corrosion inhibitors. The invention also concerns methods of preparation and use of the compositions and methods for inhibiting or preventing the corrosion of metal surfaces using said compositions, particularly in oil and gas field applications.

## Description

### Field of the Invention

The present invention relates to a composition that is suitable for use as a corrosion inhibitor to prevent corrosion, particularly on metallic devices for the recovery and transportation of hydrocarbons in oil production and processing. In particular, the present invention relates to quaternary ammonium compositions found to be effective corrosion inhibitors. The invention also concerns methods of preparation and use of the compositions and methods for inhibiting or preventing the corrosion of metal surfaces using said compositions, particularly in oil and gas field applications.

### State of the art

The exploitation of a petroleum deposit involves recovery, transport and processing operations of the hydrocarbons fluid (oil and raw natural gas). During these operations, the hydrocarbon mixture may be associated with other liquid and gas substances that expose the metallic surfaces of the devices used therein to severe corrosion damages, affecting the structure of the facilities and, consequently, being a potential cause of safety issues. Corroding substances comprise, among others, water with high salt contents, acidic gases such as carbon dioxide or hydrogen sulfide, natural organic acids and oxygen.

The state of the art discloses several ways to mitigate corrosion. One possibility is using corrosion resistant alloys, although these are rather expensive. Another possibility is the use of sacrificial anodes (cathodic protection), which are however not useful for internal corrosion control due to rapid erosion. Oxygen and H₂S scavengers are also known, although they are not effective to eliminate CO₂.

In oil recovery, transport and processing lines, corrosion is mainly anaerobic, and the so-called film-forming corrosion inhibitors (FFCIs) are mostly used. These compounds prevent corrosion by creating a layer that protects the metallic surface from being in contact with the corrosive compounds. Many different FFCIs have been investigated, including small molecules, large polymers and surfactant type compounds. However, most of the effective FFCIs compositions disclosed in the state of the art are considered to be hazardous for the environment since their acute toxicity may persist after discharge. The development of efficient environmentally friendly corrosion inhibitors is thus of high interest, due to the increasing concern of the society and authorities as to environment protection.

There are a great number of attempts described in the state of the art to provide biodegradable corrosion inhibitors, suitable to protect metal surfaces in contact with oil-based liquid compositions. The disclosed chemistries include betaines, amphoterics, modified imidazolines, phosphate esters and polyaspartate polymers. Furthermore, quaternary ammonium compounds containing ester functionalities, so-called esterquats, have been used for this purpose.

US20060201676A1 discloses a method of inhibiting corrosion during a subterranean treatment operation comprising using an acidic treatment fluid comprising an acidic fluid and an esterquat. Examples include reverse esterquats: [bis(2-hydroxyethyl)coco-betaine]cinnamyl alcohol esterquat and [bis(2-hydroxy-esthyl)coco betaine] glycerol reverse esterquat.

DE10134226 discloses a method for inhibiting corrosion on conveyors and transport devices for hydrocarbons in crude oil transport and processing, whereby an inhibitor containing a doubly alkoxylated quaternary ammonium compound is added. Quaternary ammonium compounds derived from alkoxylated amine ether carboxylic acids (being alkoxylated at least with 6 EO mols) are specifically mentioned.

DE10307730 discloses a method for inhibiting corrosion and gas hydrate formation in a mixture of hydrocarbon and water, said method comprising adding to the mixture a quaternary ammonium compound obtainable by reacting ether carboxylic acid derivatives with alkoxylated alkylamines, alkylamino alkyleneamines or alkylamino alkyletheramines to give ether carboxylic esters or ether carboxamides that are subsequently quaternised.

DE102007041216 discloses compositions that can be used to inhibit corrosion containing (a) metal salts of N-acylmethionines and (b) cationic surfactants. The description of the invention refers specifically to triethanolamine di-esterquats and diethanolmethylamine di-esterquats as possible (b) components.

DE19649285 discloses a process useful for protecting metal surfaces against corrosion involving: (a) forming a corrosion inhibitor having a quaternary ammonium compound; b) combining the corrosion inhibitor with liquid aqueous, liquid non-aqueous or gaseous media; and c) contacting the corrosion inhibitor with the metal. The corrosion inhibitor used is biodegradable and shows low toxicity. It is claimed a quaternary ammonium compound having a Markush Formula including as the groups connected to the nitrogen center alkyl, hydroxyalkyl, aryl, alkylaryl, esterificated hydroxyalkyl groups optionally alkoxylated, with C8-C24 organic acids and quaternary ammonium linked through an alkyl chain or through a dimeric acid. The authors indicate quaternary compounds having two diester groups as particularly preferred. Specifically disclosed compounds comprise methyl-N,N-bis-(coco-oxyethyl)-N-(2-hydroxyethyl) ammonium methosulfate; N,N,N-trimethyl-N-(cocooxyethyl)ammonium methosulfate; N,N-dimethyl-N-benzyl-N-(cocooxyethyl) ammonium chloride; N,N,N-trimethyl-N-(palmoxyethyl)ammonium methosulfate and N,N-dimethyl-N-benzyl-N-(palmoxyethyl) ammonium chloride.

WO2010128313 discloses a corrosion inhibitor for the oil and gas exploration, recovery and processing industries composition comprising alkoxylated (preferably ethoxylated) quaternary ammonium compounds useful for protecting a metal surface against corrosion, and in a downhole application, in a wellbore application, such as an oil drilling application, or subterranean application. The quaternary ammonium compounds structure contain at least three alkoxide units, preferably ethylene oxide units, and particularly preferred being more than 40 mol % of monoester compound, even more than 75 mol % monoester.

The present invention provides a biodegradable corrosion inhibitor based on a specific quaternary ammonium composition showing an excellent performance while being biodegradable, optionally combined with a particular family of surfactants having a synergistic effect as to the corrosion inhibitor performance.

### Summary of the invention

According to a first aspect the present invention provides a quaternary ammonium composition suitable as corrosion inhibitor comprising at least a compound of Formula (I) and at least one compound of Formula (II) or (III), being 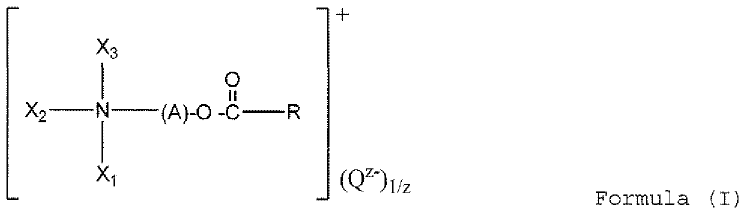 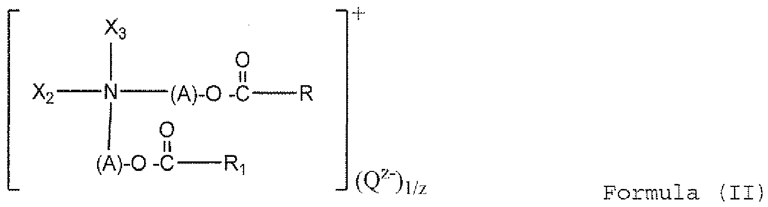 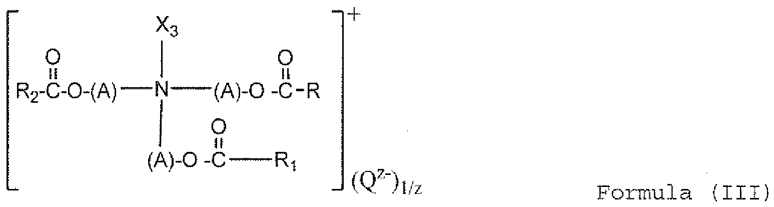 wherein
X₁ and X₂ are independently an alkyl or hydroxyalkyl group containing 1-4 carbon atoms;
X₃ is an hydroxyalkyl group containing 1-4 carbon atoms or an alkyl group containing 1-4 carbon atoms or an alkyl group containing an aromatic group.
A is an alkylene group with 2 or 3 carbon atoms, preferably a is CH₂-CH₂ group,
R, R₁ and R₂ are independently an alkyl group containing from 5 to 23 carbons atoms or an alkenyl group containing from 7 to 23 carbons atoms and 1, 2 or 3 double bonds;
Q^{z-} is an anion of charge -z where z is 1, 2 or 3.
wherein in said composition the weight ratio of the weight compounds of Formula (I) to the total weight of compounds of Formula (II) and (III) is at least 1.0.

In a second aspect, the present invention provides a method of preparing a quaternary ammonium composition according to the invention.

In a third aspect, the present invention provides a corrosion inhibitor composition comprising a quaternary ammonium compound according to the invention, preferably comprising also an ethoxylated surfactant.

In a fourth aspect the present invention provides a method of inhibiting or preventing corrosion of a metal materials comprising contacting this materials with a corrosion inhibitor composition according to the invention according to the invention, as hereinbefore defined.

### Detailed description of the invention

### 1. The Quaternary Ammonium composition

The present invention provides a composition suitable for use as corrosion inhibitor comprising a quaternary ammonium composition comprising at least a compound of Formula (I) and at least one compound of Formula (II) or (III), being 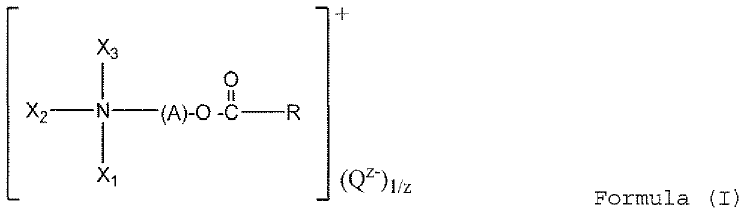 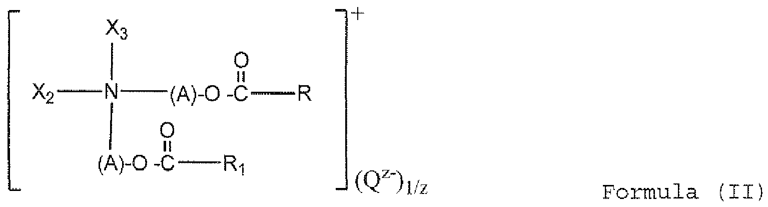 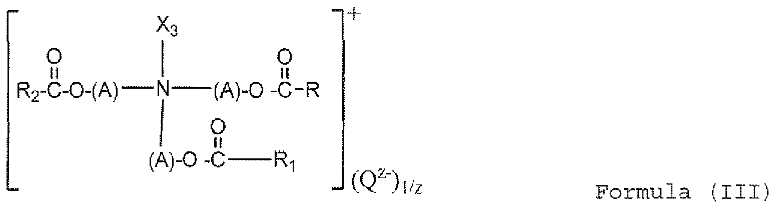 wherein
X₁ and X₂ are independently an alkyl or hydroxyalkyl group containing 1-4 carbon atoms; preferably at least one of X₁ and X₂ is a hydroxyalkyl group, more preferably X₂ and X₂ are both a hydroxyalkyl group, even more preferred X₁ and X₂ are CH₂CH₂OH.
X₃ is an hydroxyalkyl group containing 1-4 carbon atoms or an alkyl group containing 1-4 carbon atoms or an alkyl group containing one aromatic group, preferably X₃ is an alkyl group, more preferably X₃ is a methyl group.
A is an alkylene group with 2 or 3 carbon atoms, preferably a is CH₂-CH₂ group,
R, R₁ and R₂ are independently a linear or branched alkyl group containing 5-23 carbons atoms or a linear or branched alkenyl group containing 7-23 carbons atoms and 1, 2 or 3 double bonds;
Q^{z-} is an anion of charge -z where z is 1, 2 or 3.

In said composition, the ratio of the weight of compounds of Formula (I) to the total weight of compounds of Formula (II) and (III) is at least 1.0, preferably at least 1.2, more preferably at least 1.5, even more preferably at least 1.8, most preferred 2.0. Furthermore, it is preferred that the ratio of the weight of compounds of Formula (I) to the total weight of compounds of Formula (II) and (III) is 5 or less, more preferably 4 or less, particularly preferred being 3 or less.

According to the invention, the counter-anion Q^{z-} in Formula (I) is an halide or an alkylsulphate. Preferably the counter-ion is chloride or methyl sulfate.

In a preferred embodiment the composition comprises alkylated alkanolamine of Formula (V), e.g. methylated triethanolamine.

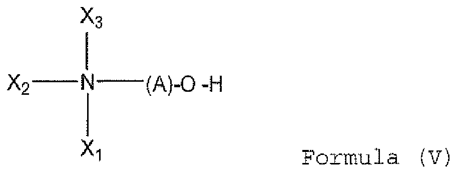

In a specially preferred embodiment, the weight ratio of the total weight of compounds of Formula (I), (II) and (III) to the compound of Formula (V) is lower than 15:1, more preferably lower than 10:1.

The relative concentration of species of Formula (I), (II), (III) and (V) can be determined according to standard chromatographic and NMR methods known by the skilled in the art.

In a preferred embodiment, the quaternary ammonium composition according to the invention comprises the compounds of Formula (I), (II), (III) and (V) together with a solvent. In a particularly preferred embodiment the quaternary ammonium composition according to the invention comprises the compounds of Formula (I), (II), (III) and (V) in a total amount of 50 to 99.5 wt.% (more preferred 60 to 95 wt.%, even more preferred 70 to 90 wt.) and the solvent in an amount of 0.5 to 50 wt.% (more preferred 5 to 40 wt.%, even more preferred 10 to 30 wt.), the total amount of the compounds of Formula (I), (II), (III) and (V) and solvent being at least 80 wt.%, preferably at least 90 wt.%, and most preferably at least 95 wt.% with respect to the total amount of the quaternary ammonium composition. The solvent is preferably water or one or more C1-C6 alcohols, such as isopropanol, ethyleneglycol, propyleneglycol, butyldiglycol or mixtures thereof. Preferably, the solvent is butylglycol. The quaternary ammonium composition of the present invention may also be provided without a solvent particularly if used in a corrosion inhibitor composition containing a liquid carrier, as discussed below.

### 2. The method of preparing a quaternary ammonium composition according to the invention

In a second aspect, the present invention provides a method of preparing a quaternary ammonium composition according to the invention.

The quaternary ammonium composition according to the invention as hereinbefore defined can be prepared by reacting a fatty acid with an alkanolamine to obtain a mixture containing an esteramine, and subsequently quaternising the mixture with an alkylating agent.
In particular, the quaternary ammonium composition according to the invention is obtainable by a process comprising the following steps.

First, the fatty acid undergoes condensation (esterification) with the alkanolamine.

### The fatty acid

The fatty acid is a product obtained from oils and fats from plants and animals, such as palm, sunflower, soybean, palm olein, olive, canola, tall oil, tallow, etc.., possibly totally or partially hydrogenated and purified, or synthetic fatty acids such as palmitoleic acid, oleic acid, elaidinic acid, petroselinic acid, linoleic acid, linolenic acid, gadoleic acid, behenic acid and erucic acid, or mixtures thereof. Preferably the fatty acid comprises one ore more compounds of Formula RₓCOOH, where Rₓ is an alkyl or alkenyl, linear or branched unsaturated chain, having from 6 to 24 carbon atoms, more preferably from 8 to 22 carbon atoms. In a more preferred embodiment, the fatty acid comprises as main (more abundant) fatty acid a compound of Formula RCOOH where Rₓ is an alkyl or alkenyl, linear or branched unsaturated chain, having from 12 to 22 carbon atoms, more preferably from 14 to 18 carbon atoms. In a most preferred embodiment, the main (more abundant) fatty acid is oleic acid.

The preferred embodiments of Rx define the preferable structures of R, R1 and R2 in Formula (I), (II) and (III).

In a preferred embodiment, the quaternary ammonium composition according to the invention is obtained from fatty acids having an iodine number of from 0 to 250, preferably from 50 to 250, more preferably from 80 to 120. A particularly preferred fatty acid is oleic acid.

### The alkanolamine

The alkanolamine is a compound of Formula (IV) 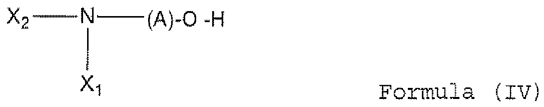 wherein A is an alkylene group with 2 or 3 carbon atoms, preferably A is CH₂-CH₂ group,
X₁ and X₂ are independently an alkyl or hydroxyalkyl group containing 1-4 carbon atoms; preferably at least one of X₁ and X₂ is a hydroxyalkyl group, more preferably X₁ and X₂ are both a hydroxyalkyl group, even more preferred X₁ and X₂ are CH₂CH₂OH.

Examples of alkanolamines of Formula (IV) suitable to produce a composition according to the invention are triethanolamine, methyldiethanolamine, ethyldiethanolamine, dimethylethanolamine, diethylethanolamine.

In a preferred embodiment, the alkanolamine is triethanolamine, methyldiethanolamine or dimethylethanolamine or mixtures thereof, more preferably triethanolamine.

### The esterification conditions

The esterification can be conducted in a known way, as described for example in patent application ES-A-2021900. Preferably the esterification reaction is effected by condensation of fatty acid with alkanolamine (e.g. triethanolamine) at a temperature between 120° C and 220° C, for a period of 2-10 hours, preferably at a reduced pressure of about 5 to 200 mbar and in the presence of one of the catalysts known for the esterification, such as hypophosphorous acid or paratoluenesulfonic acid, and also in the presence of any of the usual stabilizers and antioxidants such as tocopherols, BHT, BHA, etc. To obtain an esteramine mixture suitable as precursor of the quaternary ammonium composition according to the invention, it is important to control the fatty acid / alkanolamine molar ratio during the esterification step. Preferably, the fatty acid / alkanolamine molar ratio is lower than 1.2, in order to ensure that the amount of monoester compound is at suitable levels according to the invention.

The product resulting from the esterification reaction comprises a mixture of mono-, di-and tri-esters of fatty acids, free alkanolamine of Formula (IV) and free fatty acid.

### The quaternization

The quaternisation of the esterification reaction product of alkanolamine with the fatty acid is conducted in known way, as described for example in WO-A-9101295. Preferred alkylating agents include methyl chloride, dimethyl sulfate or mixtures thereof.

The product according to the present invention is obtainable by using a molar ratio of alkylating agent on the total esteramine that generally ranges from 0.6 to 1.0, preferably from 0.8 to 1.0.

The quaternization can be carried at temperatures between 55 ° C and 120 0 ° C. The composition that results from the quaternization process comprises quaternised ester compounds having one (monoesterquat), two (diesterquat) or three (triesterquat) ester groups, and quaternised alkanolamine. Also, the product can contain small amounts of the unreacted fatty acid.

### Particularly preferred quaternary ammonium composition

In a particularly preferred embodiment, the quaternary ammonium composition of the present invention is obtained by esterification of triethanolamine and a C16 and/or C18 fatty acid, particularly oleic acid. In this embodiment, the ratio of the weight of compounds of Formula (I) to the total weight of compounds of Formula (II) and (III) is in the range of 2.0 to 3.0. The total amount of the compounds of Formula (I), (II), (III) and (V) in is in the range of 70 to 95 wt%, and a solvent is present in an amount of 5 to 30 wt.%.

### 3. The corrosion inhibitor composition

A corrosion inhibitor composition according to the invention is a composition suitable to prevent corrosion of a material, in particular the corrosion of a metal or an alloy, when added to a liquid or gas media in contact with said material.

In a third aspect, the present invention provides a corrosion inhibitor composition comprising a quaternary ammonium composition according to the invention, preferably comprising also one or more cosurfactants comprising and alkoxylated surfactant. In a preferred embodiment, these co-surfactants are either carboxylic acids or carboxylic acid esters.

In a preferred embodiment, the weight ratio of the quaternary ammonium composition according to the invention and the co-surfactant is from 100:1 to 0,5:1, preferably from 50:1 to 1:1, more preferably from 20:1 to 2:1, even more preferably from 10:1 to 3:1.

In a preferred embodiment, the preferred corrosion inhibitor compositions of the invention comprise a co-surfactant comprising one or more alkyl ether carboxylic acids or its salts, and/or one or more polyethoxylated glycerine esters or mixtures thereof.

### The alkyl ether carboxylic acids or its salts

In a first particularly preferred embodiment of the invention, the quaternary ammonium composition according to the invention is provided in combination with one or more ether carboxylic acid or its salt of Formula (V_{I}):

R₅-(OCH₂CH₂)ₚ-OCH₂CH₂C (=O)-O M Formula (VI)

wherein R₅ is a linear or branched alkyl chain having from 4 to 30 carbon atoms,
wherein p is 0 or an integer of 1 to 30, and
wherein M is H or a cation.

Ether carboxylates of Formula (VI) are products well known in the art. They can be obtained by alkoxylation and subsequent carboxymethylation of fatty alcohols as described by Meijer and Smid in Palyethex Carboxylates; Anionic Surfactants; Surfactant Sciencie Series, Vol. 56 (p. 313-361), edited by Helmut W. Stache, ISBN: 0-8247-9394-3.

The general process to obtain ether carboxylates comprises two steps.

The first one is the alkoxylation of alcohols under standard conditions known by the skilled in the art. For instance, the polyoxyethylene group is obtained by addition of ethylene oxide to fatty alcohols, mostly with an alkaline catalyst such as NaOH, KOH or NaOCH₃, giving a broad polyoxyethylene oxide distribution (broad ethoxylation degree). For special applications the employed catalyst can be a narrow range catalyst suitable to obtain a narrow distribution of ethoxylated products, such as Lewis acids or metallic Na or NaH. However, one may also start from commercially available ethoxylated alcohols.

The second step is the carboxymethylation of the ethoxylated alcohols by means of a process that comprise the reaction of the alkoxylated alcohol with a strong base, like sodium or potassium hydroxide, in presence of a reducing agent, eg. sodium borohydride, to obtain the corresponding alkoxylate, which is carboxymethylated with sodium monochloroacetate (SMCA).

The ether carboxylates of Formula (VI) are derived from C₄-C₃₀ fatty alcohols, which are preferably derived from natural fat and oil as well as of synthetic origin. Preferred fats and oils include palm oil, coconut oil, sunflower oil, rapeseed oil, castor oil, olive oil, soybean oil; and animal fat such as tallow, bone oil; fish oil, hardened oils and semihardened oils thereof; and mixtures thereof. As a result of its natural origin, the C₄-C₃₀ fatty alcohols that are alkoxylated and subsequently carboxymethylated may contain a great variety of alkyl and/or alkenyl groups, said groups being linear or branched, saturated or unsaturated.

Particularly preferred are C₄-C₃₀ fatty alcohols which are derived from coconut oil, palm oil, palm-kernel, olive oil as well as synthetic origin.

According to the invention, it is preferred that in the ether carboxylates of Formula (VI) p has a value in the range of 1 to 20, preferably in the range of 1 to 15, more preferably in the range 4 to 15. Preferably, M is hydrogen, sodium, potassium or a quaternary ammonium compound.

In a preferred embodiment it is also preferred that in the C₄-C₃₀ alkyl and/or alkenyl group in the ether carboxylates of Formula (VI) the C₁₂-C₁₄ proportion is higher than 80%, more preferred higher than 80%, even more preferred higher than 85%.

Examples of commercially available ether carboxylates of Formula (VI) are AKYPO ^{®} RO 10 VG (Oleyl ether carboxylic acid with an average ethoxylation degree of 1), AKYPO ^{®} RO 20 VG (Oleyl ether carboxylic acid with an average ethoxylation degree of 2), AKYPO ^{®} RO 50 VG (Oleyl ether carboxylic acid with an average ethoxylation degree of 5), and AKYPO ^{®} RO 90 VG (Oleyl ether carboxylic acid with an average ethoxylation degree of 9), AKYPO ^{®} RLM 100 (Lauryl ether carboxylic acid with an average ethoxylation degree of 11), AKYPO ^{®} RLM 45 (Lauryl ether carboxylic acid with an average ethoxylation degree of 4), AKYPO ^{®} LF6 (Capryl/Butyl ether carboxylic acid with an average ethoxylation degree of 9/2), AKYPO ^{®} LF2 (Octyl ether carboxylic acid with an average ethoxylation degree of 9), all marketed by KAO Chemicals Europe.

### The polyethoxylated glycerine esters

In one particularly preferred embodiment of the invention the quaternary ammonium composition according with the invention is provided in combination with one or more polyethoxylated glycerine ester compounds. Preferably, the polyethoxylated glycerine ester composition comprises a mixture of compounds of Formula (VII): 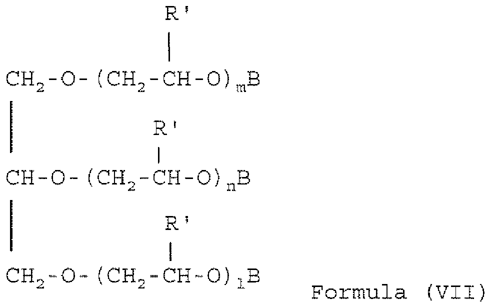 wherein each one of m, n, or 1 represents, independently, a number of 0 to 40, the sum of m, n and 1 being in the range of 1 to 200, B being a hydrogen atom or an acyl group represented by -CO-R', R' representing an alkyl or alkenyl group, linear or branched, with 3 to 21 carbon atoms, preferably with 5 to 17 carbon atoms, more preferably with 5 to 11 carbon atoms, wherein the mixture comprises the following compounds i. to iv. :
i. at least one component represented by Formula (VII), wherein, independently, one of the groups B represents an acyl group represented by -CO-R' and the remaining ones represent H
ii. at least one component represented by Formula (VII), wherein, independently, two of the groups B represent an acyl group represented by -CO-R' and the remaining one represents H;
iii. at least one component represented by Formula (VII), wherein, independently, each one of the groups B represents an acyl group represented by - CO-R';
iv. at least one component represented by Formula (VII), wherein each one of groups B represents H.

Such mixtures of alkoxylated glycerides and alkoxylated glycerine can be prepared by using the preparation methods as described in the European patent applications EP-A-0579887, EP-A-0586323, EP-A-1045021, and EP-A-2029711B1 and are commercially available under the trademark LEVENOL^{®} and marketed by Kao Chemicals Europe

In a preferred embodiment the proportion by weight of the species (i)/(ii)/(iii) is in the range 46-90/9-35/1-15

In a further preferred embodiment the proportion by weight (i) + (ii) + (iii) / (iv) is in the range of 3.0:0.3 to 0.5:3.0.

Further preferred compositions of the invention comprise a surfactant and or other ingredients known to show corrosion inhibition properties. These ingredients include alkyl quaternary amines, other types of alkyl esterquats, alkyl amido quats, alkyl amido amines, alkyl imidazolines, alkyl imidazoline derivatives, including amphoacetates, quaternised and non-quaternised alkyl amines, alkyl amides, alkyl sarcosinates and alkyl glutamates, alkyl betaines and alkyl amido betaines, sulfo-betaines, alkyl imino propionates, beta alanines, alkyl phosphonates and / or mono or bis phosphate esters, carboxylates such as carboxymethosuccinic compounds, aminohydroxysuccinic compounds and oligomers or polymers of tartaric acid, polyaminoacids such as polyaspartic acid, polyaspartates (optionally functionalized with amino or thiol group)or alkylpolyglucosides.

The compositions of the present invention may also include wetting agents, stability aids, agents to increase or decrease the partition of components between phases, demulsifiers, or antifoaming agents. Examples of suitable ingredients include sulfosuccinates, alkyl ether carboxylates (or salts), alkyl ether alcohols, ethoxylated fatty acid esters, ethoxylated fatty acid amines and ethoxylated fatty acid amides.

### The liquid carrier

In one embodiment of the invention the corrosion inhibitor composition according to the invention comprises a carrier, particularly a liquid carrier. The carrier would be used whenever the composition properties, specially those in connection with the physical state of the ingredients of such a compositon, should be adapted in order to enable its performance as corrosion inhibitor. In particular, the carrier could be used in order the corrosion inhibitor composition is a solution or a dispersion, wherein the dissolved or dispersed materials would comprise the quaternary ammonium compounds according to the invention.

It is envisaged that the compositions may also be in different forms, i.e. they may be oil-miscible, water-miscible, oil-water dispersions, water-oil dispersions or present in the form of an emulsion of any type. Preferably the composition is in the form of a solution.

The liquid carrier may be aqueous (e.g. water) or non-aqueous, but is preferably non-aqueous. Preferred non-aqueous carriers include C₁₋₈ alcohols such as methanol, ethanol, isopropanol, isobutanol, hexanol or 2-ethyl hexanol; glycols such as ethyleneglycol, di-ethyleneglycol, propyleneglycol, ethyl diglycol, butyldiglycol, dipropylene glycol, ethylene glycol monobutyl ether (EGMBE) and the like, or mixtures thereof. Other suitable carriers include hydrocarbon distillates, fatty acid esters and carbonic acid esters, phenyl esters, C₉₋₁₂ aromatics and turpenes. Preferably the carrier is butyl diglycol or butyl glycol.

The skilled person will readily be able to determine a suitable amount of carrier. This amount is that amount which allows the ingredients of the corrosion inhibitor composition to be introduced in the media which is in contact with the material to be protected from corrosion in such a way that the corrosion inhibitor composition efficiently protects the material. The amount of carrier is preferably present from 0.5 to 99.5 weight %, more preferably between 5 and 60 weight %, even more preferably between 40 and 60 weight %, with respect to the total amount of the corrosion inhibitor composition.

The corrosion inhibitor compositions of the present invention may also comprise other additives known in the art for use in well treatment. In sweet systems, for instance, the compounds of the invention may be used in combination with a sulphur containing species such as a thiosulphate salt, 2-mercaptoethanol, thioglycolic acid or alkyl thiols. Some preferred compositions therefore comprise a sulphur containing species such as a thiosulphate salt, 2-mercaptoethanal, thioglycolic acid or alkyl thiols. Particularly preferred compositions comprise sodium thiosulphate. When present, the weight ratio of the quaternary ammonium composition according to the present invention to the sulfur containing species should be from 100 to 0.1, more preferably from 20 to 0.1, most preferably from 10 to 1.

Most generally, the corrosion inhibitor compositions of the present invention comprise the quaternary ammonium compounds, and preferably the alkoxylated cosurfactants according to the invention, are designed to mitigate primarily corrosion. However, it can also be envisaged that the quaternary ammonium compositions of the present invention, which are useful as corrosion inhibitors, may be used in a multi-functional composition where the quaternary ammonium compounds according to the invention are used in combination with other agents designed to reduce, for example, scale formation, gas hydrate formation, wax or paraffin formation or to mitigate any other problem related to the recovery, processing and transportation of hydrocarbons. Thus corrosion inhibitor compositions of the present invention may be not only intended to inhibit corrosion but to work to improve other aspects of the oil production and transport process, and in this regard they also may include additives such as scale inhibitors, gas hydrate inhibitors, wax inhibitors, paraffin inhibitors, thickeners, diversion agents, pH modifiers and/or catalysts.

### 4. A method of inhibiting or preventing corrosion of a metal materials

In a fourth aspect the present invention provides a method of inhibiting or preventing corrosion of a metal materials comprising contacting this materials with a corrosion inhibitor composition according to the invention, as hereinbefore defined

In the following, some details relating to the process used to inhibit corrosion according the invention and in particular the application of compositions of the present invention as a corrosion inhibitor composition in oil and gas field applications are described.

In the process to inhibit corrosion in the oil field operation equipments according to the invention, the corrosion inhibitor composition comprising a quaternary ammonium composition according to invention is added to the medium contacting the equipment in effective quantities. Preferably, the concentration of the added corrosion inhibitor composition is between 0 and 100 ppm, based on the weight percentage in the media, preferably between 0 and 50 ppm.

The composition to inhibit corrosion according the invention is preferably used to protect metal surfaces of the equipment (pipelines, valves, tubes, etc) used in oil recovery operations. However, it can be used to protect metal surfaces not only in oil recovery, but also in oil transport and processing operations.

For the purpose of the present invention, the media to which the metals are exposed may be liquid or gas, this is, liquid/liquid or liquid/gaseous multiphase system. The liquid phase can be purely aqueous, non-aqueous (for example crude oil) and, more often, a water/oil mixture or emulsion. In general, the compositions of the present invention may be applied to any system in which corrosion is likely to occur. This includes any water-containing system, e.g. boilers, coolers, scrubbers, heat transfer systems, fluid processing vessels and transport pipes. Preferably, the system is a hydrocarbon producing system, refinery system or other system containing both water and hydrocarbon. Most preferably, the system is a hydrocarbon production and separation system or hydrocarbon pipeline system.

Within such a system, the media to which the metallic surfaces are exposed may be a liquid or a gas, including liquid/liquid or liquid/gaseous multiphase systems. The liquid phase can be purely aqueous, non-aqueous (for example crude oil) and, more often, a water/oil mixture or emulsion. The media may to which the metallic surfaces are exposed may also be a slurry.

In order to address these different conditions, the form in which the compositions of the present invention are deployed may be varied. In particular, the compositions of the invention may be designed with different characteristics, i.e. they may be oil-miscible, water-miscible, oil-water dispersions, water-oil dispersions or as an emulsion of any type.

The corrosion inhibitor compositions of the present invention may also be used alone or in combination with other agents.

The proportions of the ingredients described above with reference to the corrosion inhibitor composition according to the invention that may be included in the compositions and the amount of the composition that is required to inhibit corrosion, or to reduce it to an acceptable level, varies according to the system in which it is used. Methods for assessing and monitoring the effectiveness of treatments in different systems are well known by those skilled in the art. Most generally, the compositions of the invention must be added to the system at an appropriate concentration. The composition must also be added at a point in the system that is upstream of the locations at which corrosion inhibition is required.

It can be envisaged that compositions of the present invention may be deployed by several different techniques. These include, but are not limited to: (i) placement into a sump, cavity or annulus; (ii) placement into a pipe or pipeline, e.g. as part of a hydrotest operation; (iii) by continuous injection into a pipe or pipeline e.g. via a pump; (iv) by batch treatment, including the treatment of equipment and pipelines and (v) by direct treatment of valuable devices (fittings, valves, tubes, pipelines, heat exchangers etc.).

By illustration, when used for treatment into a sump, cavity or annulus, when used for treatment by a 'batch' process, or when placed into a pipe or pipeline as part of a hydrotest operation, the effective concentration of a composition, according to the invention can range from 0.01 to 50% w/w concentration, more preferably from 0.1 to 15% w/w concentration and more preferably still from 0.1 to 50, based on the total volume of the sump, cavity, annulus or pipeline.

When used for continuous injection into a pipe or pipeline via a pump, the effective concentration of composition according to the invention can range from 1 to about 5000 parts per million by weight (ppm), more preferably from 10 to about 1000 ppm and more preferably still from 10 to about 250 ppm, based on total fluids and also taking into account the flow rate of the main fluid stream. In this illustration, treatment by continuous injection includes treatment, e.g. into flow lines of any sizes, gathering lines, flow lines, by-pass loops and headers.

Preferably the treatment methods of inhibiting or preventing corrosion of the present invention are applied to a hydrocarbon producing system. The term "hydrocarbon producing system" is used herein to encompass the subterranean formation (e.g. rock) from which hydrocarbon is extracted as well as the equipment used in the extraction process. The formation may contain oil or gas, although the method is particularly suitable for treatment of oil wells. The metal surface may be the surface of any equipment used, including subsurface and surface equipment (e.g. tubes, pipes, pumps, valves, nozzles, storage containers, screens etc). In a preferred aspect of the present invention the corrosion of hydrocarbon extraction and transportation equipment is inhibited or prevented using the compounds and compositions hereinbefore defined.

The following examples are given in order to provide a person skilled in the art with a sufficiently clear and complete explanation of the present invention, but should not be considered as limiting of the essential aspects of its subject, as set out in the preceding portions of this description.

### Examples

The first part of the Examples section refers to the preparation of the quarternary ammonium compositions according to the invention and to the preparation of comparative examples.

The second part of the Examples section refers to performance of corrosion inhibitor compositions according to the invention and to the performance of comparative examples of them.

### 1. Preparation of the quaternary ammonium compositions according to the invention.

### Example A

### Esterification

377 grams of Oleic acid were introduced in an inert atmosphere into a stainless steel reactor, and 251g of triethanolamine were added with stirring. The mixture was heated for at least 4 hours at 160-180°C in order to remove the water of the reaction. The progress of the reaction was monitored by an acid/base assay which determines the residual acidity to obtain an esterification of at least 90 to 95% of the fatty acids.

606 g of a yellowish liquid product, consisting essentially of a mixture of unesterified fatty acids, mono-, di- and triesterified amine and unreacted triethanolamine may also remain.

### Quaternization

202.1g of dimethyl sulphate were added with stirring at a temperature of 40-90°C to 606g of the product obtained from the esterification step and diluted in 200g of butyldiglycol. After two hours of digestion, the virtually complete absence of residual amine was verified by acid/base assay. 1008 g of quaternized esteramine 2 diluted in butyldiglycol were obtained.

The composition of the final product is determined with a High Performanace Liquid Chromatography (HPLC)using a Strong Cation Exchange Column and an Evaporative Light Scattering detector and 1H Nuclear Magnetic Ressonance (1H-NMR)at 500 MHz (signals between 3.25 and 3.38).

The weight ratio of the weight of the compounds of Formula (I) to the total weight of the compounds of Formulae (II) and (III) is 2.2.

### Examples B-E

The quaternary ammonium compositions where prepared analogously to Example A, although modifying the amounts reactants ( triethanolamine, oleic acid and dimethylsulfate) according to the data in Table 1.

**Table 1**

| Example | Oleic acid (g) | Triethanolamine (g) | Dimethyl sulphate (g) | Weight ratio (I)/((II)+(III) * |
|---|---|---|---|---|
| B | 316 | 278.3 | 224.1 | 2.9 |
| C | 410 | 216.6 | 174.5 | 1.6 |
| D | 480 | 195 | 157.2 | 0.8 |
| E | 526 | 173.7 | 134.0 | 0.4 |

| | | | | |
|---|---|---|---|---|
| (*) This weight ratio corresponds to the ratio of species of Formula (I) to the total of species of Formulae (II) and/or (III). | | | | |

Quaternary ammonium compositions A, B and C are according to the invention whereas D and E are comparative examples. In the following section this quaternary ammonium compositions are used as ingredients of corrosion inhibitor compositions according to the invention.

### 2. Corrosion Inhibition assays

### Examples

The performance of the compositions according to the invention, and the performance of comparative examples, as to corrosion inhibition, was tested by means of linear polarisation resistance (LPR) measurements.

A corrosion system is emulated in a laboratory cell equipped with LPR prove with steel carbon electrodes. Corrosion medium is a bi-phase system composed of a brine phase (8% total dissolved solids) and an oily phase (white spirit) in a ratio brine/oil 4:1. The medium is submitted to constant CO₂ stream and stirring. Once the system is stabilized, 20 ppm of Corrosion Inhibitor Formula (equals to 4.8 ppm of active quaternary ammonium compound) is added to the cell, by being dropped to the upper phase which is oil. The corrosion inhibitor composition in weight percentages is: Quaternary ammonium composition 30%; cosurfactant (if present) 5%; Water (19%), Sodium Thiosulphate (1%), Butylglycol (added up to 100%).

LPR is periodically measured by automatic means. Thus, a corrosion intensity v.s. time table is obtained. The % inhibition can be calculated using the Formula: % inhibition = 100 100*final corrosion rate/initial corrosion rate, measured after one hour and after three hours.

Table 2 details the corrosion inhibitor compositions tested corresponding to compositions according to the invention (1-8) and comparative examples (C1-C5).

Tables 3 details the results of the corrosion inhibition tests for compositions as detailed in Table 1. As can be seen, the compositions according with the invention provide higher and faster protection.

**Table 2.**

| Experiment Code | Quaternary Ammonium composition | Cosurfactant (1) |
|---|---|---|
| 1 | A | None |
| 2 | B | None |
| 3 | B | LEVENOL N-661 |
| 4 | A | LEVENOL N-661 |
| 5 | B | AKYPO RLM 100 |
| 6 | A | AKYPO RLM 100 |
| 7 | C | LEVENOL N-661 |
| 8 | C | AKYPO RLM-100 |
| C1 | D | None |
| C2 | E | None |
| C3 | D | LEVENOL N-661 |
| C4 | E | LEVENOL N-661 |
| C5 | E | AKYPO RLM 100 |

The chemical names of the cosurfactants are as follows:
AKYPO RLM 100 - Polyoxyethylene(10) lauryl ether carboxylic acid
LEVENOL N-661 - Polyoxyethylene C₈₋₁₀ glycerides

**Table 3.**

| Experiment code | Inhibition (%) measured after 1 hour | Inhibition (%) measured after 3 hours |
|---|---|---|
| 1 | 76 | 96 |
| 2 | 56 | 93 |
| 3 | 98 | 99 |
| 4 | 98 | 98 |
| 5 | 97 | 98 |
| 6 | 97 | 98 |
| 7 | 89 | 96 |
| 8 | 81 | 98 |
| C1 | 2 | 9 |
| C2 | 5 | 28 |
| C3 | 2 | 13 |
| C4 | 1 | 5 |
| C5 | 0 | 7 |

The results in Table 3 show that the corrosion inhibitor compositions comprising a quaternary ammonium composition according to the invention allow a nearly complete protection against corrosion. Besides, the presence of the cosurfactant significantly accelerates the protection.

## Claims

1. A quaternary ammonium composition comprising at least a compound of Formula (I) and at least one compound of Formula (II) or (III), being 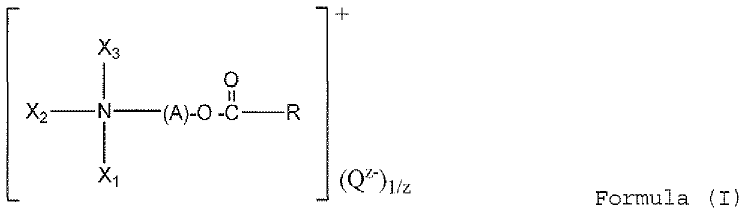 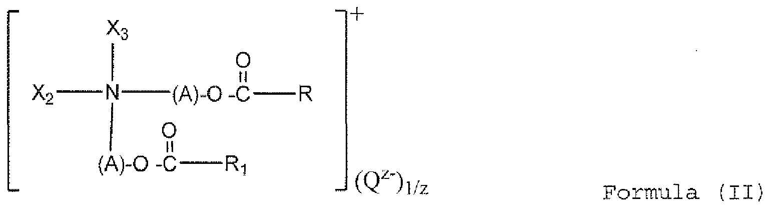 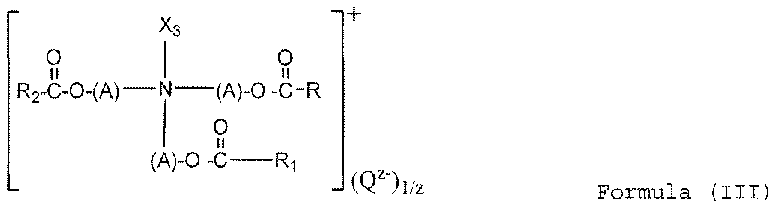 wherein
X₁ and X₂ are independently an alkyl or hydroxyalkyl group containing 1-4 carbon atoms;
X₃ is an hydroxyalkyl group containing 1-4 carbon atoms or an alkyl group containing 1-4 carbon atoms or an alkyl group containing an aromatic group.
A is an alkylene group with 2 or 3 carbon atoms, preferably a CH₂-CH₂ group,
R, R₁ and R₂ are independently a linear or branched alkyl group containing 5-23 carbons atoms or a linear or branched alkenyl group containing 7-23 carbons atoms and 1, 2 or 3 double bonds;
Q^{z-} is an anion of charge -z where z is 1, 2 or 3.
wherein in said composition the weight ratio of the weight of the compounds of Formula (I) to the total weight of the compounds of Formulae (II) and (III) is at least 1.0, preferably at least 1.2, more preferably at least 1.5, even more preferably at least 1.8, most preferred being at least 2.0.

2. The composition according to claim 1, wherein the composition is obtainable by reacting a fatty acid with an alkanolamine to obtain a mixture containing an esteramine, and subsequently quaternising the mixture with an alkylating agent.

3. The composition according to claim 2, wherein the fatty acid is a fatty acid having an iodine number of 0 to 250, more preferably from 50 to 250, even more preferably 80-120.

4. The composition according to claim 3, wherein the fatty acid is oleic acid.

5. The composition according to one or more of claims 2 to 4, wherein the alkanolamine is triethanolamine, methyldiethanolamine or dimethylethanolamine or mixtures thereof.

6. The composition according to one or more of claims 1 to 5 additionally comprising one or more alkoxylated co-surfactants.

7. A composition according to claim 6 wherein the alkoxylated co-surfactant is an ether carboxylic acid or its salt of Formula (VI)
R₅-(OCH₂CH₂)ₚ-OCH₂CH₂C (=O) -O M Formula (VI)
wherein R₅ is a linear or branched alkyl chain having from 4 to 30 carbon atoms,
wherein p is 0 or an integer of 1 to 30, preferably 1 to 20, more preferably in the range of 1 to 15, and
wherein M is H or a cation, preferably hydrogen, sodium, potassium or a quaternary ammonium compound.

8. The composition according to claim 6 wherein the alkoxylated surfactant is a polyethoxylated glycerine ester composition.

9. The composition according to claim 8 wherein the polyethoxylated glycerine ester composition is a mixture of compounds of Formula (VII): 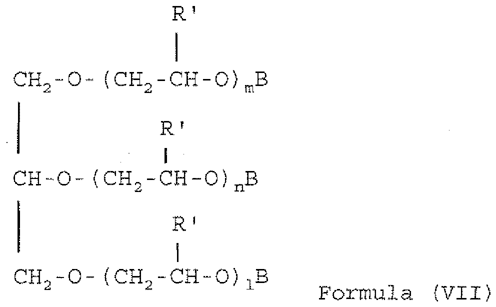 wherein each one of m, n, or 1 represents, independently, a number of 0 to 40, the sum of m, n and 1 being in the range of 1 to 200, B being a hydrogen atom or an acyl group represented by -CO-R', R' representing an alkyl or alkenyl group, linear or branched, with 3 to 21 carbon atoms, preferably with 5 to 17 carbon atoms, more preferably with 5 to 11 carbon atoms, wherein the mixture comprises the following compounds i. to iv. :
i. at least one component represented by Formula (VII), wherein, independently, one of the groups B represents an acyl group represented by -CO-R' and the remaining ones represent H
ii. at least one component represented by Formula (VII), wherein, independently, two of the groups B represent an acyl group represented by -CO-R' and the remaining one represents H;
iii. at least one component represented by Formula (VII), wherein, independently, each one of the groups B represents an acyl group represented by - CO-R';
iv. at least one component represented by Formula (VII), wherein each one of groups B represents H.

10. The composition according to claim 9 wherein the proportion by weight (i) + (ii) + (iii) / (iv) is in the range of 3.0:0.3 to 0.5:3.0.

11. The composition according to one or more of claims 1 to 10 that further comprise a carrier, particularly a liquid carrier.

12. The composition according to one or more of claims 1 to 11 that further comprises one or more solvents.

13. The composition according to one or more of claims 1 to 12 is provided in combination with a thiosulphate salt.

14. Use of the composition according to one or more of claims 1 to 13 for inhibiting corrosion.

15. A method to inhibit corrosion, said method comprising adding a composition according to one or more of claims 1 to 15 to a metal surface.
